# FASCICULE DE BREVET EUROPEEN

(11) **EP 3 946 164 B1**
(45) Date de publication et mention de la délivrance du brevet: **07.06.2023**
(21) Numéro de dépôt: 20731527.6
(22) Date de dépôt: 20.03.2020
(51) Int. Cl.: A61F 2/26

(54) **SYSTÈME PROTHÉTIQUE À POMPE HYDRAULIQUE MOTORISÉE ADAPTÉ POUR CONTRÔLER LE GONFLEMENT D'UN ÉLÉMENT GONFLABLE**
PROTHESENSYSTEM MIT MOTORISIERTER HYDRAULIKPUMPE ZUR STEUERUNG DES AUFBLASENS EINES AUFBLASBAREN ELEMENTS
PROSTHETIC SYSTEM WITH MOTORIZED HYDRAULIC PUMP DESIGNED TO CONTROL THE INFLATION OF AN INFLATABLE ELEMENT

(30) Priorité: 02.04.2019 FR 1903527
(43) Date de publication de la demande: 09.02.2022
(73) Titulaire: Zephyr Surgical Implants, 51370 Saint-Brice-Courcelles (FR)
(72) Inventeur: GOMEZ-LLORENS, Christophe, 51370 Saint-Brice-Courcelles (FR)
(74) Mandataire: Cabinet Beau de Loménie
(86) Numéro de dépôt international: PCT/FR2020/050606
(87) Numéro de publication internationale: WO 2020/201660

(56) Documents cités:
- WO-A2-01/47439
- WO-A2-2009/094431
- US-A- 5 823 991
- US-A1- 2007 142 700

## Description

### Domaine Technique

La présente invention concerne le domaine technique des systèmes prothétiques ou d'implants chirurgicaux à pompe hydraulique motorisée, adaptés pour contrôler le gonflement d'un élément gonflable en réponse à une pression d'un fluide. Plus précisément, l'objet de l'invention concerne les systèmes prothétiques ou d'implants chirurgicaux à pompe hydraulique motorisée avec transfert d'énergie sans fil.

La présente invention trouve une application préférée pour un implant pénien pour créer une érection. La présente invention trouve d'autres applications pour constituer un sphincter au sens général tel un sphincter oesophagien ou un anneau gastrique ou un sphincter urinaire ou anal.

### Technique antérieure

Dans le domaine technique des systèmes prothétiques destinés à lutter contre l'impuissance masculine, l'art antérieur a proposé diverses solutions prothétiques. Par exemple, les documents EP 1 255 514 et WO 01/47439, WO 2009/094431 décrivent différentes variantes de réalisation d'un système prothétique comportant un dispositif implanté dans le corps d'un patient et un boîtier de commande extérieur au corps du patient et intégrant un système de transfert d'énergie. Par exemple, le dispositif implanté dans le corps du patient comporte un implant pénien gonflable en réponse à une pression d'un fluide contenu dans un réservoir et déplacé à l'aide d'une pompe motorisée alimentée en énergie par le système de transfert d'énergie sans fil. Un tel système comporte également un dispositif implantable dans le patient pour assurer le dégonflage de l'implant pénien. Il ressort qu'un tel système n'offre pas une totale sûreté en ce qui concerne notamment l'opération de dégonflage de l'implant. De plus, le dispositif implanté dans le patient comporte différentes fonctions ne permettant pas de les intégrer dans un boîtier peu encombrant.

Il est également connu par le document US 2007/142700 un système prothétique avec un dispositif implanté comprenant au moins un élément gonflable en réponse à une pression d'un fluide, en communication avec un réservoir contenant ce fluide et une pompe assurant la circulation du fluide. Le système implanté demande notamment pour l'action de gonflage une action mécanique de l'utilisateur sur une pompe manuelle. La mise en oeuvre d'un tel système s'avère compliquée en pratique pour certains patients.

La présente invention vise à remédier aux inconvénients des dispositifs antérieurs en proposant un nouveau système prothétique à pompe hydraulique motorisée, adapté pour contrôler le gonflement d'un élément gonflable, un tel système présentant une totale sécurité de fonctionnement aussi bien pour les opérations de gonflage que pour les opérations de dégonflage de l'élément gonflable.

Un autre objet de l'invention est de proposer un nouveau système prothétique dont le dispositif implanté dans le corps d'un patient est conçu pour présenter un encombrement limité.

### Exposé de l'invention

Pour atteindre un tel objectif, l'objet de l'invention vise un système prothétique hydraulique comportant un dispositif implanté dans le corps d'un patient et un boîtier de commande extérieur au corps du patient ;
* le dispositif implanté comportant :
   - au moins un élément gonflable en réponse à une pression d'un fluide ;
   - un réservoir pour le fluide en communication avec l'élément gonflable ;
   - un boîtier étanche dans lequel est montée une pompe motorisée par un moteur électrique alimenté en énergie par un système de transfert d'énergie sans fil dont une partie est montée dans le boîtier, cette pompe étant en communication d'une part avec l'élément gonflable via un obturateur unidirectionnel de refoulement et, d'autre part, avec le réservoir via un obturateur unidirectionnel d'aspiration, la pompe assurant uniquement la circulation du fluide du réservoir vers l'élément gonflable pour assurer son gonflage ;
   - une commande manuelle de dégonflage de l'élément gonflable accessible de l'extérieur du boîtier étanche et agissant sur l'obturateur unidirectionnel d'aspiration et sur l'obturateur unidirectionnel de refoulement pour ouvrir la communication entre l'élément gonflable et le réservoir lors de son actionnement manuel ;
* le boîtier de commande intégrant une autre partie du système de transfert d'énergie et assurant de piloter le fonctionnement du système de transfert d'énergie.

Le système selon l'invention peut avantageusement être mis en oeuvre avec l'une et / ou l'autre des caractéristiques additionnelles suivantes :
- la pompe motorisée comporte un soufflet délimitant une chambre pour le fluide dont le volume varie sous l'action du moteur électrique, cette chambre communiquant avec l'élément gonflable via l'obturateur unidirectionnel de refoulement et avec le réservoir via l'obturateur unidirectionnel d'aspiration ;
- le moteur électrique agit sur le soufflet via un système de transformation du mouvement de rotation du moteur électrique en un mouvement de translation assurant la compression et la dilatation de la chambre pour le fluide ;
- le système de transformation du mouvement de rotation du moteur électrique en un mouvement de translation comporte un jeu de galets entraînés en rotation par le rotor du moteur électrique et coopérant avec une came fixée au soufflet ;
- chaque obturateur unidirectionnel d'aspiration et de refoulement est rappelé élastiquement à sa position fermée de repos ;
- chaque obturateur unidirectionnel d'aspiration et de refoulement comporte un clapet sollicité par un ressort en appui sur un siège présenté par le boîtier, les clapets étant portés par une tige d'actionnement dont une extrémité libre est accessible de l'extérieur du boîtier pour constituer la commande manuelle de dégonflage de l'élément gonflable ;
- le système de transfert d'énergie sans fil comporte une antenne émettrice intégrée au boîtier de commande et une antenne réceptrice montée dans le boîtier étanche et reliée à une carte électronique de conversion en un courant continu alimentant le moteur électrique ;
- le boîtier de commande comporte un bouton de mise en marche du système de transfert d'énergie sans fil et une temporisation du fonctionnement du système de transfert d'énergie sans fil au terme d'une durée déterminée d'émission ;
- au moins un élément gonflable est un implant pénien.

### Brève description des dessins

[Fig. 1] La Figure 1 est une vue en perspective illustrant un exemple de réalisation d'un système prothétique d'implant pénien.
[Fig. 2] La Figure 2 est une vue en coupe élévation montrant un boîtier intégrant une pompe motorisée faisant partie du système prothétique conforme à l'invention.
[Fig. 3] La Figure 3 est une vue en perspective en éclaté montrant un exemple de réalisation préféré de la pompe motorisée montée dans le boîtier illustré à la Fig. 2.
[Fig. 4] La Figure 4 est une vue en coupe élévation analogue à la Fig. 2 et montrant les obturateurs unidirectionnels d'aspiration et de refoulement de la pompe, occupant une position d'ouverture.
[Fig. 5] La Figure 5 est une vue en coupe élévation du boîtier analogue à la Fig. 2 et montrant la commande manuelle de dégonflage de l'élément gonflable en position de dégonflage.

### Description des modes de réalisation

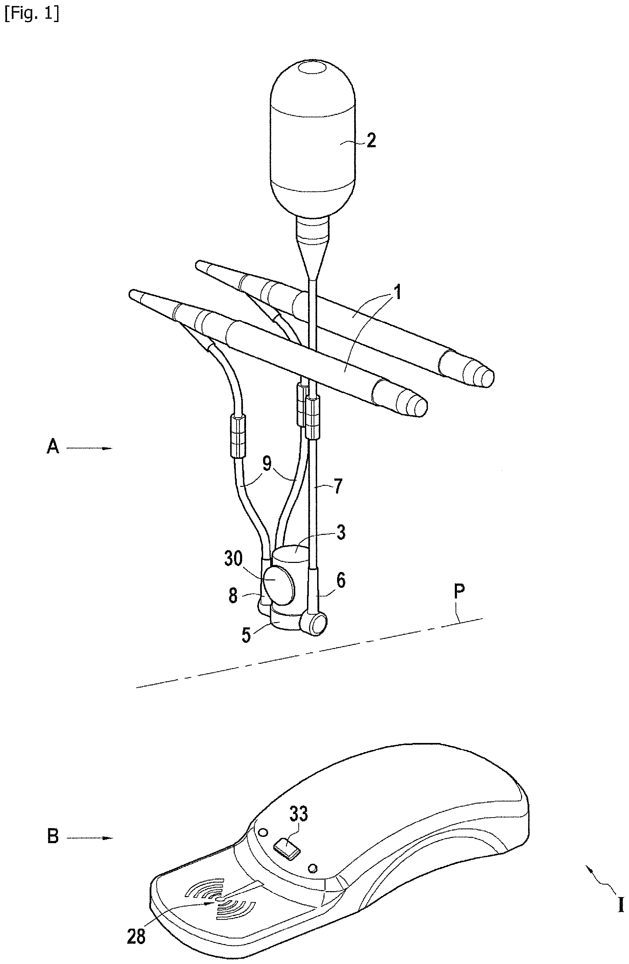

Tel que cela ressort plus précisément de la Fig. 1, l'objet de l'invention concerne un système I prothétique ou d'implants chirurgicaux à pompe hydraulique motorisée avec transfert d'énergie sans fil. Ce système prothétique I comporte un dispositif A destiné à être implanté dans le corps du patient et un boîtier de commande B extérieur au corps du patient. Un trait mixte P schématise la peau du patient pour montrer que le dispositif A est implanté dans le corps du patient alors que le boîtier de commande B reste à l'extérieur du corps du patient.

Le dispositif implanté A comporte au moins un et dans l'exemple illustré, deux éléments gonflables 1 en réponse à une pression d'un fluide. Selon cet exemple préféré de réalisation, le système prothétique 1 vise à créer un implant pénien pour créer une érection. Selon cette application, les deux éléments gonflables 1 se présentent chacun sous la forme d'un corps allongé destiné à être placé dans les corps caverneux du pénis d'un homme et destiné à occuper une position droite d'érection lors du gonflage des éléments gonflables 1 et une position repliée lorsque les éléments gonflables 1 sont dégonflés.

Bien entendu, l'élément gonflable 1 peut présenter des formes différentes en fonction des applications visées. Ainsi, l'élément gonflable 1 peut se présenter sous la forme d'une manchette apte à entourer un organe pourvu d'un passage interne destiné à être fermé ou ouvert par constriction par cet élément gonflable. Par exemple, cet élément gonflable peut être un sphincter oesophagien, un anneau gastrique ou un sphincter urinaire ou anal.

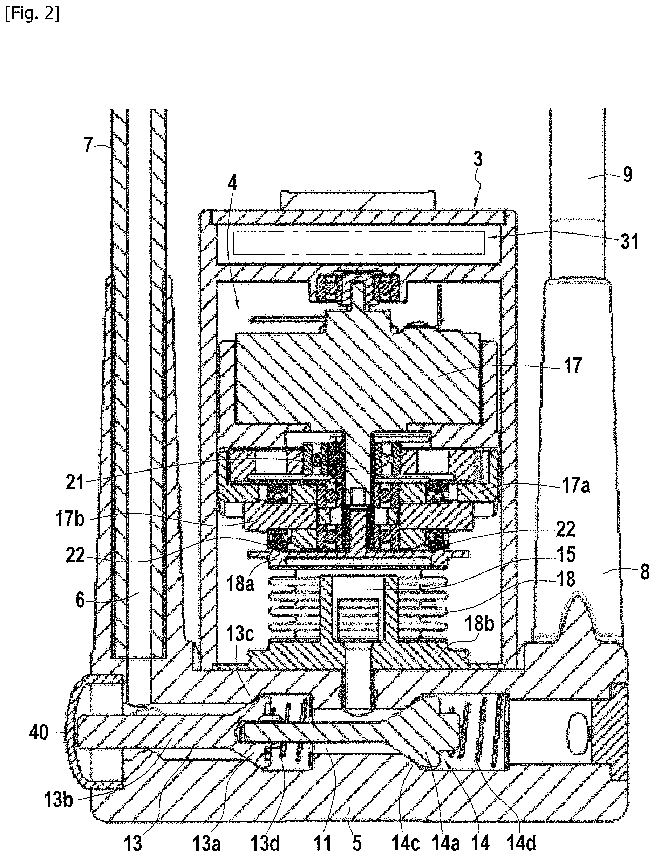

Le dispositif implanté A comporte également un réservoir 2 pour le fluide, connecté pour être en communication avec les éléments gonflables 1. Le dispositif implanté A comporte également un boîtier étanche 3 dans lequel est montée une pompe motorisée 4 conçue pour assurer la circulation du fluide uniquement du réservoir 2 vers les éléments gonflables 1 (Fig. 2). Ce boîtier 3 qui présente un caractère étanche comporte une tête de pompe 5 pourvue d'un côté, d'une tubulure 6 de raccordement via un tuyau 7 au réservoir 2 et d'un autre côté, d'une tubulure 8 de communication via des conduits, 9 à chaque élément gonflable 1.

Tel que cela ressort plus précisément de la Fig. 2, la tête de la pompe 5 comporte un conduit 11 de circulation pour le fluide communiquant d'un côté, avec la tubulure 6 de raccordement au réservoir 2 et de l'autre côté, avec la tubulure 8 de communication avec les éléments gonflables 1. Le conduit 11 est aménagé dans la tête de pompe 5 en étant équipé d'un obturateur unidirectionnel d'aspiration 13 disposé entre la pompe motorisée 4 et la tubulure 6 de raccordement avec le réservoir 2. Le conduit 11 est équipé également d'un obturateur unidirectionnel de refoulement 14 interposé entre la pompe motorisée 4 et la tubulure 8 de communication avec les éléments gonflables 1.

Tel que cela ressort des dessins, chaque obturateur unidirectionnel d'aspiration 13 et de refoulement 14 est rappelé élastiquement à sa position fermée de repos. Selon une variante préférée de réalisation, l'obturateur unidirectionnel d'aspiration 13 comporte un clapet 13a porté par une tige 13b montée à l'intérieur du conduit 11. Le clapet 13a est destiné à coopérer sous l'action d'un ressort de rappel 13d, avec un siège 13c aménagé dans la tête de pompe 5. De même, l'obturateur unidirectionnel de refoulement 14 comporte un clapet 14a porté par la tige 13b et destiné à coopérer avec un siège 14c aménagé dans la tête de la pompe 5. Le clapet 14a est sollicité en appui sur le siège 14c par un ressort de rappel 14d.

Le conduit 11 communique entre l'obturateur unidirectionnel d'aspiration 13 et l'obturateur unidirectionnel de refoulement 14, avec une chambre 15 pour le fluide dont le volume varie sous l'action de la pompe motorisée 4. Cette chambre 15 communique ainsi avec les éléments gonflables 1 via l'obturateur unidirectionnel de refoulement 14 et avec le réservoir 2, via l'obturateur unidirectionnel d'aspiration 13. La pompe motorisée 4 est conçue pour assurer uniquement l'aspiration du fluide du réservoir 2 en vue de l'amener dans les éléments gonflables 1. Typiquement, le fluide utilisé est du liquide physiologique.

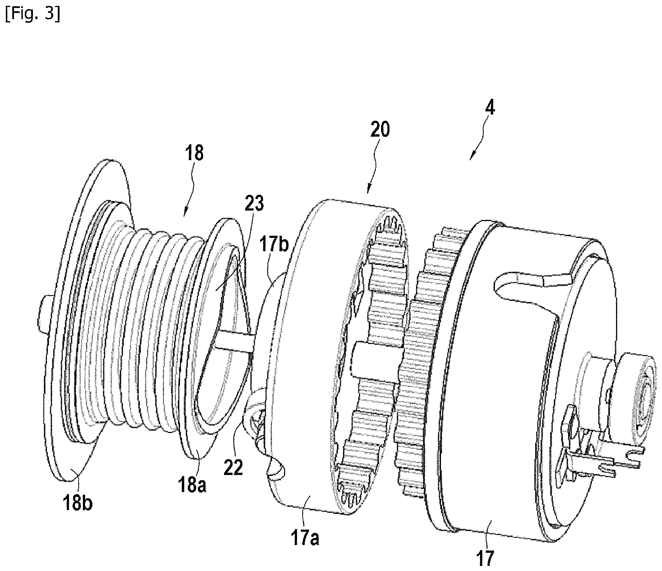

Selon une variante préférée de réalisation illustrée plus particulièrement à la Fig. 3, cette pompe motorisée 4 comporte un moteur électrique 17 agissant sur un soufflet 18 délimitant intérieurement, la chambre 15 pour le fluide à volume variable. Le moteur électrique 17 agit sur le soufflet 18 via un système 20 de transformation du mouvement de rotation du moteur électrique 17 en un mouvement de translation assurant la compression et la dilatation du soufflet permettant de faire varier le volume de la chambre 15 pour le fluide. Par exemple, le moteur électrique 17 est un motoréducteur électrique comportant un moteur électrique à courant continu à balais intégrant un réducteur entraînant en rotation un rotor 17a autour d'un axe de rotation 21. Il est à noter que le moteur électrique 17 est dimensionné pour ne pas atteindre la pression d'éclatement des éléments gonflables 1. Ainsi, lorsque l'élément gonflable 1 a atteint sa pression de gonflage, le moteur 17 cale en cas de poursuite de sa commande de fonctionnement, la pression d'éclatement des éléments gonflables étant trois fois supérieure à la pression de gonflage maximale. Il n'y a aucun risque d'éclatement. De plus, au bout d'une temporisation prédéfinie et légèrement supérieure au temps de gonflage des éléments gonflables, l'alimentation électrique du moteur 17 est coupée. Par exemple, le moteur 17 présente une puissance de 1.2W.

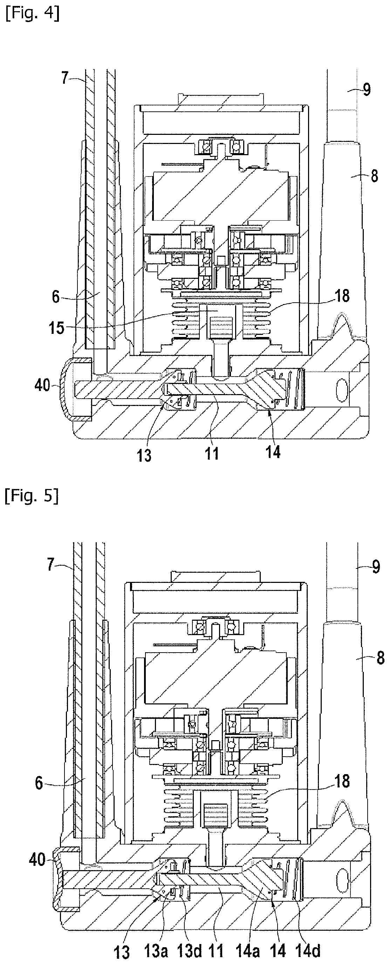

Le rotor 17a transmet son mouvement de rotation au système de transformation 20 du mouvement de rotation du moteur électrique 17 en un mouvement de translation. Ce système de transformation 20 comporte un jeu de deux galets 22 entraînés en rotation par le rotor 17a du moteur électrique et coopérant avec une came 23 fixée au soufflet. Dans l'exemple illustré, le rotor 17a comporte une plaque de fond 17b munie des deux galets 22 disposés symétriquement de part et d'autre de l'axe de rotation 21, avec leurs axes de roulement perpendiculaires à l'axe de rotation 21 du rotor. Les galets 22 sont destinés à coopérer avec un chemin d'une came 23 aménagée sur un flasque mobile 18a du soufflet 18 présentant un flasque fixe 18b fixé sur la tête de pompe 5. La came 23 possède un profil adapté de manière que le roulement des galets 22 sur le chemin de came conduit à l'application d'un effort symétrique de part et d'autre de l'axe de symétrie du soufflet 18 de manière que ce dernier se trouve comprimé ou dilaté successivement lors de la rotation du rotor. Cette came 23 permet à l'aide du moteur, de créer un mouvement alternatif de compression du soufflet 18, et donc de pompage tel qu'illustré à la figure 4 pour lequel s'ouvrent l'obturateur unidirectionnel d'aspiration 13 et l'obturateur unidirectionnel de refoulement 14. Le soufflet 18 possède une élasticité adaptée pour permettre la variation de la chambre 15 pour assurer le pompage. Le soufflet 18 qui est réalisé dans des métaux implantables assure une barrière étanche entre le fluide hydraulique et l'ensemble constitué par le système de transformation de mouvement 20 et le moteur électrique 17. Le pompage est réalisé sans frottement, uniquement par déformation élastique du soufflet. Il est à noter que la pompe 4 assure la circulation du fluide uniquement du réservoir en direction des éléments gonflables 1 grâce à la configuration des obturateurs unidirectionnels d'aspiration et de refoulement.

Selon une caractéristique de l'invention, le moteur électrique 17 est alimenté en énergie par un système de transfert d'énergie sans fil dont une partie est montée dans le boîtier 3 tandis qu'une autre partie du système de transfert d'énergie est montée dans le boîtier de commande B. Selon une caractéristique préférée de réalisation, le système de transfert d'énergie sans fil comporte une bobine ou antenne émettrice 28 intégrée au boîtier de commande B et une antenne ou bobine réceptrice 30 montée dans le boîtier étanche 3 implanté dans le corps du patient. Cette antenne réceptrice 30 est reliée à une carte électronique 31 montée dans le boîtier 3 et permettant de transformer le courant haute fréquence de l'antenne réceptrice 30 en un courant continu alimentant le moteur électrique 17. Comme cela ressort plus précisément de la Fig. 1, l'antenne réceptrice 30 est montée latéralement sur le boîtier 3 pour permettre un couplage optimum avec l'antenne émettrice 28. Bien entendu, l'antenne réceptrice 30 est montée de manière étanche à l'intérieur du boîtier 3. Ainsi, le boîtier étanche 3 implanté dans le corps du patient ne comporte pas de batterie.

Le boîtier de commande B comporte un bouton 33 de mise en marche du système de transfert d'énergie sans fil permettant le transfert d'énergie à l'antenne réceptrice 30 montée dans le boîtier étanche 3. Avantageusement, ce bouton 33 active le système de transfert d'énergie tant qu'il est activé manuellement. Ce boîtier de commande B comporte une temporisation du fonctionnement du système de transfert d'énergie sans fil au terme d'une durée déterminée d'émission. Typiquement, le système de transfert d'énergie sans fil interrompt son émission après une durée d'émission continue par exemple de 3 mn, même si une action perdure sur le bouton 33.

Selon une caractéristique de l'invention, le dispositif implanté A comporte une commande manuelle 40 de dégonflage des éléments gonflables 1, accessible de l'extérieur du boîtier étanche 3. Cette commande manuelle 40 agit à la fois sur l'obturateur unidirectionnel d'aspiration 13 et sur l'obturateur unidirectionnel de refoulement 14 pour ouvrir la communication entre les éléments gonflables 1 et le réservoir 2. Dans l'exemple de réalisation, la commande manuelle 40 est réalisée par un bouton de dégonflage aménagé sur la tête 5 de la pompe et constitué par une membrane souple montée pour fermer de manière étanche le conduit 11. Cette commande manuelle 40 permet par un appui, l'actionnement de la tige 13b supportant les obturateurs unidirectionnels 13, 14 de manière à assurer leur ouverture. Avantageusement, l'appui sur la commande manuelle 40 conduit au déplacement simultané de l'obturateur unidirectionnel d'aspiration 13 et de l'obturateur unidirectionnel de refoulement 14. Tel que cela ressort de la figure 5, les clapets 13a, 14a sont déplacés à l'encontre des ressorts 13d, 14d conduisant les clapets à ne plus coopérer avec leur siège autorisant ainsi le fluide à circuler des éléments gonflables 1 en direction du réservoir 2. Cette commande manuelle 40 permet un dégonflage à coup sûr des éléments gonflables 1.

Le fonctionnement du système prothétique hydraulique I conforme à l'invention découle de la description qui précède. Le boîtier de commande B est approché de la peau P du patient au-dessus de l'endroit où est implanté le boîtier étanche 3. La mise à proximité de l'antenne émettrice 28 avec l'antenne réceptrice 30 permet d'optimiser le couplage. L'actionnement du bouton 33 du boîtier de commande B active le système de transfert d'énergie conduisant à l'alimentation électrique du moteur 17 de la pompe. Il s'ensuit que la pompe 4 assure le transfert du fluide du réservoir 2 jusqu'aux éléments gonflables 1. Lorsque les éléments gonflables 1 sont gonflés, le bouton 33 est relâché. Pour le dégonflage des éléments gonflables 1, un appui sur la commande manuelle 40 permet de placer simultanément l'obturateur unidirectionnel d'aspiration 13 et l'obturateur unidirectionnel de refoulement 14 en position d'ouverture assurant le retour du fluide dans le réservoir 2.

L'invention n'est pas limitée aux exemples décrits et représentés car diverses modifications peuvent y être apportées sans sortir de son cadre.

## Revendications

1. Système prothétique hydraulique comportant un dispositif implanté (A) dans le corps d'un patient et un boîtier de commande (B) extérieur au corps du patient ;
* le dispositif implanté comportant :
- au moins un élément gonflable (1) en réponse à une pression d'un fluide ;
- un réservoir (2) pour le fluide en communication avec l'élément gonflable ;
- un boîtier étanche (3) dans lequel est montée une pompe motorisée (4) par un moteur électrique (17) alimenté en énergie par un système de transfert d'énergie sans fil dont une partie (30) est montée dans le boîtier, cette pompe étant en communication d'une part avec l'élément gonflable via un obturateur unidirectionnel de refoulement (14) et, d'autre part, avec le réservoir (2) via un obturateur unidirectionnel d'aspiration (13), la pompe assurant uniquement la circulation du fluide du réservoir (2) vers l'élément gonflable (1) pour assurer son gonflage ;
- une commande manuelle (40) de dégonflage de l'élément gonflable (1) accessible de l'extérieur du boîtier étanche (3) et agissant sur l'obturateur unidirectionnel d'aspiration (13) et sur l'obturateur unidirectionnel de refoulement (14) pour ouvrir la communication entre l'élément gonflable (1) et le réservoir lors de son actionnement manuel ;
* le boîtier de commande (B) intégrant une autre partie (28) du système de transfert d'énergie et assurant de piloter le fonctionnement du système de transfert d'énergie.

2. Système selon la revendication 1, selon lequel la pompe motorisée (4) comporte un soufflet (18) délimitant une chambre (15) pour le fluide dont le volume varie sous l'action du moteur électrique, cette chambre (15) communiquant avec l'élément gonflable (1) via l'obturateur unidirectionnel de refoulement (14) et avec le réservoir (2) via l'obturateur unidirectionnel d'aspiration (13).

3. Système selon la revendication 2, selon lequel le moteur électrique (17) agit sur le soufflet (18) via un système (20) de transformation du mouvement de rotation du moteur électrique en un mouvement de translation assurant la compression et la dilatation de la chambre pour le fluide.

4. Système selon la revendication 3, selon lequel le système de transformation (20) du mouvement de rotation du moteur électrique (17) en un mouvement de translation comporte un jeu de galets (22) entraînés en rotation par le rotor du moteur électrique et coopérant avec une came (23) fixée au soufflet (18).

5. Système selon l'une des revendications précédentes, selon lequel chaque obturateur unidirectionnel d'aspiration (13) et de refoulement (14) est rappelé élastiquement à sa position fermée de repos.

6. Système selon la revendication précédente, selon lequel chaque obturateur unidirectionnel d'aspiration (13) et de refoulement (14) comporte un clapet (13a,14a) sollicité par un ressort (13d,14d) en appui sur un siège (13c,14d) présenté par le boîtier, les clapets étant portés par une tige d'actionnement (13b) dont une extrémité libre est accessible de l'extérieur du boîtier pour constituer la commande manuelle (40) de dégonflage de l'élément gonflable (1).

7. Système selon la revendication précédente, selon lequel le système de transfert d'énergie sans fil comporte une antenne émettrice (28) intégrée au boîtier de commande (B) et une antenne réceptrice (30) montée dans le boîtier étanche (3) et reliée à une carte électronique (31) de conversion en un courant continu alimentant le moteur électrique.

8. Système selon l'une des revendications précédentes, selon lequel le boîtier de commande (B) comporte un bouton (33) de mise en marche du système de transfert d'énergie sans fil et une temporisation du fonctionnement du système de transfert d'énergie sans fil au terme d'une durée déterminée d'émission.

9. Système selon l'une des revendications précédentes, selon lequel au moins un élément gonflable (1) est un implant pénien.

## Patentansprüche

1. Hydraulisches Prothesensystem, umfassend eine in den Körper eines Patienten implantierte Vorrichtung (A) und ein Steuergehäuse (B) außerhalb des Körpers des Patienten;
* wobei die implantierte Vorrichtung umfasst:
- wenigstens ein als Reaktion auf einen Druck eines Fluids aufblasbares Element (1);
- einen Behälter (2) für das Fluid in Verbindung mit dem aufblasbaren Element;
- ein dichtes Gehäuse (3), in dem eine Pumpe (4) angebracht ist, die durch einen Elektromotor (17), welcher von einem drahtlosen Energieübertragungssystem, von dem ein Teil (30) in dem Gehäuse angebracht ist, mit Energie versorgt wird, motorisch angetrieben wird, wobei diese Pumpe einerseits mit dem aufblasbaren Element über einen unidirektionalen Druckverschluss (14) und andererseits mit dem Behälter (2) über einen unidirektionalen Saugverschluss (13) in Verbindung steht, wobei die Pumpe lediglich das Zirkulieren des Fluids von dem Behälter (2) zu dem aufblasbaren Element (1) sicherstellt, um dessen Aufblasen sicherzustellen;
- eine Handbedienung (40) zum Entleeren des aufblasbaren Elements (1), die von außerhalb des dichten Gehäuses (3) zugänglich ist und auf den unidirektionalen Saugverschluss (13) und auf den unidirektionalen Druckverschluss (14) einwirkt, um die Verbindung zwischen dem aufblasbaren Element (1) und dem Behälter während seiner manuellen Betätigung zu öffnen;
* wobei das Steuergehäuse (B) einen weiteren Teil (28) des Energieübertragungssystems enthält und sicherstellt, dass der Betrieb des Energieübertragungssystems gesteuert wird.

2. System nach Anspruch 1, bei dem die motorisch angetriebene Pumpe (4) einen Faltenbalg (18) aufweist, der eine Kammer (15) für das Fluid begrenzt, deren Volumen unter der Wirkung des Elektromotors variiert, wobei diese Kammer (15) mit dem aufblasbaren Element (1) über den unidirektionalen Druckverschluss (14) und mit dem Behälter (2) über den unidirektionalen Saugverschluss (13) in Verbindung steht.

3. System nach Anspruch 2, bei dem der Elektromotor (17) auf den Faltenbalg (18) über ein System (20) zum Umwandeln der Rotationsbewegung des Elektromotors in eine Translationsbewegung wirkt, das das Komprimieren und das Ausdehnen der Kammer für das Fluid sicherstellt.

4. System nach Anspruch 3, bei dem das System (20) zum Umwandeln der Rotationsbewegung des Elektromotors (17) in eine Translationsbewegung einen Satz von Rollen (22) umfasst, die durch den Rotor des Elektromotors drehangetrieben werden und mit einem an dem Faltenbalg (18) befestigten Nocken (23) zusammenwirken.

5. System nach einem der vorhergehenden Ansprüche, bei dem jeder unidirektionale Saug- (13) und Druckverschluss (14) in seine geschlossene Ruheposition elastisch zurückgestellt wird.

6. System nach dem vorhergehenden Anspruch, bei dem jeder unidirektionale Saug- (13) und Druckverschluss (14) eine Klappe (13a, 14a) umfasst, die durch eine Feder (13d, 14d) in Anlage an einen durch das Gehäuse dargestellten Sitz (13c, 14d) belastet ist, wobei die Klappen von einer Betätigungsstange (13b) getragen sind, von welcher ein freies Ende von außerhalb des Gehäuses zugänglich ist, um die Handbedienung (40) zum Entleeren des aufblasbaren Elements (1) zu bilden.

7. System nach dem vorhergehenden Anspruch, bei dem das drahtlose Energieübertragungssystem eine Sendeantenne (28), die in das Steuergehäuse (B) integriert ist, sowie eine Empfangsantenne (30) umfasst, die in dem dichten Gehäuse (3) angebracht und mit einer elektronischen Karte (31) zur Umwandlung in den Elektromotor speisenden Gleichstrom verbunden ist.

8. System nach einem der vorhergehenden Ansprüche, bei dem das Steuergehäuse (B) einen Knopf (33) zum Starten des drahtlosen Energieübertragungssystems und eine Abschaltverzögerung für den Betrieb des drahtlosen Energieübertragungssystems am Ende einer bestimmten Sendedauer umfasst.

9. System nach einem der vorhergehenden Ansprüche, bei dem wenigstens ein aufblasbares Element (1) ein Penisimplantat ist.

## Claims

1. A hydraulic prosthetic system including a device implanted (A) in the body of a patient and a control case (B) outside the patient's body;
* the implanted device including:
- at least one inflatable element (1) that can be inflated in response to a pressure of a fluid;
- a tank (2) for the fluid in communication with the inflatable element;
- a sealed case (3) in which is mounted a motor-driven pump (4) driven by an electric motor (17) supplied with energy by a wireless energy transfer system, part (30) of which is mounted in the case, this pump being in communication on the one hand with the inflatable element via a one-way discharge obturator (14) and on the other hand with the tank (2) via a one-way suction obturator (13), the pump ensuring only the circulation of the fluid from the tank (2) to the inflatable element (1) to ensure its inflation;
- a manual command (40) for deflating the inflatable element (1) accessible from outside the sealed case (3) and acting on the one-way suction obturator (13) and on the one-way discharge obturator (14) to open the communication between the inflatable element (1) and the tank during its manual actuation;
* the control case (B) integrating another part (28) of the energy transfer system and ensuring the control of the operation of the energy transfer system.

2. The system according to claim 1, according to which the motor-driven pump (4) includes a bellows (18) delimiting a chamber (15) for the fluid whose volume varies under the action of the electric motor, this chamber (15) communicating with the inflatable element (1) via the one-way discharge obturator (14) and with the tank (2) via the one-way suction obturator (13).

3. The system according to claim 2, according to which the electric motor (17) acts on the bellows (18) via a system (20) for transforming the rotational movement of the electric motor into a translational movement ensuring the compression and the expansion of the chamber for the fluid.

4. The system according to claim 3, according to which the system for transforming (20) the rotational movement of the electric motor (17) into a translational movement includes a set of rollers (22) driven in rotation by the rotor of the electric motor and cooperating with a cam (23) fixed to the bellows (18).

5. The system according to any of the preceding claims, according to which each one-way suction (13) and discharge (14) obturator is elastically returned to its closed rest position.

6. The system according to the preceding claim, according to which each one-way suction (13) and discharge (14) obturator includes a valve (13a, 14a) biased by a spring (13d, 14d) to bear on a seat (13c, 14d) presented by the case, the valves being carried by an actuation rod (13b), one free end of which is accessible from outside the case to constitute the manual command (40) for deflating the inflatable element (1).

7. The system according to the preceding claim, according to which the wireless energy transfer system includes a transmitting antenna (28) integrated into the control case (B) and a receiving antenna (30) mounted in the sealed case (3) and connected to an electronic card (31) for conversion into a direct current supplying the electric motor.

8. The system according to any of the preceding claims, according to which the control case (B) includes a button (33) for activating the wireless energy transfer system and a timing of the operation of the wireless energy transfer system after a determined duration of transmission.

9. The system according to any of the preceding claims, wherein at least one inflatable element (1) is a penile implant.
